# EUROPÄISCHE PATENTSCHRIFT

(11) **EP 3 185 807 B1**
(45) Veröffentlichungstag und Bekanntmachung des Hinweises auf die Patenterteilung: **11.01.2023**
(21) Anmeldenummer: 15754135.0
(22) Anmeldetag: 11.08.2015
(51) Int. Cl.: A61B 34/00, A61B 1/12

(54) **VERFAHREN UND SYSTEM ZUM BETRIEB EINER AUFBEREITUNGSVORRICHTUNG FÜR CHIRUGISCHE INSTRUMENTE**
METHOD AND SYSTEM FOR OPERATING A RECONDITIONING APPARATUS FOR SURGICAL INSTRUMENTS
PROCÉDÉ ET SYSTÈME DE PRÉPARATION DES INSTRUMENTS CHIRUGICAUX

(30) Priorität: 29.08.2014 DE 102014217335
(43) Veröffentlichungstag der Anmeldung: 05.07.2017
(73) Patentinhaber: Olympus Winter & Ibe GmbH, 22045 Hamburg (DE)
(72) Erfinder: THATE, Henning, 22417 Hamburg (DE)
(74) Vertreter: Seemann & Partner Patentanwälte mbB
(86) Internationale Anmeldenummer: PCT/EP2015/068437
(87) Internationale Veröffentlichungsnummer: WO 2016/030180

(56) Entgegenhaltungen:
- DE-A1-102010 060 302
- JP-A- H06 142 041
- JP-A- 2002 272 822
- JP-A- 2006 230 491
- JP-A- 2013 106 790
- JP-B1- 5 537 752

## Beschreibung

Die Erfindung betrifft ein Verfahren und ein System zum Betrieb einer Aufbereitungsvorrichtung für chirurgische Instrumente, insbesondere Endoskope, wobei die Aufbereitungsvorrichtung eine Hauptsteuerung und wenigstens ein austauschbares Verschleißteil aufweist, das für einen Betrieb bis zu einer vorbestimmten Höchstbetriebsdauer vorgesehen ist.

Aufbereitungsvorrichtungen von chirurgischen Instrumenten, insbesondere von Endoskopen, verfügen üblicherweise über Systeme bzw. Vorrichtungen, die sowohl die äußeren Oberflächen der chirurgischen Instrumente als auch die Kanäle bzw. Kanalsysteme derselben reinigen und desinfizieren. Unter einer Aufbereitungsvorrichtung ist im Rahmen der vorliegenden Erfindung daher auch eine Reinigungs- und Desinfektionsvorrichtung für chirurgische Instrumente zu verstehen.

In entsprechenden Reinigungsvorrichtungen bzw. Aufbereitungsvorrichtungen für chirurgische Instrumente wird derzeit eine Hauptsteuerung für die Überwachung aller eingebauten Sensoren verwendet, wobei mittels der Hauptsteuerung die Reinigungsschritte gesteuert und überwacht werden. Entsprechende Aufbereitungsvorrichtungen sehen beispielsweise vor, eine Spülflüssigkeit durch Kanäle des chirurgischen Instruments, beispielsweise eines Endoskops, zu leiten, um diese Kanäle zu spülen. Zusätzlich können chemische Mittel verwendet werden, um diese zu desinfizieren. Eine Wärmeeinwirkung und eine Bestrahlung mit keimtötendem Licht, beispielsweise UV-Licht, dient ebenfalls der Reinigung und Desinfektion.

In den Aufbereitungsvorrichtungen befinden sich Bauteile, die aufgrund ihres kritischen Einsatzbereiches bezüglich ihrer Betriebsdauer bzw. Lebensdauer begrenzt sind und überwacht werden. Solche Bauteile sind beispielsweise UV-Leuchtmittel oder Filter. Für entsprechende Leuchtmittel wird im Rahmen aufwändiger Hygieneprüfungen eine Lebensdauer bzw. Höchstbetriebsdauer definiert und dann mittels eines Zählers überwacht.

Dieser Zähler befindet sich in der zugeordneten Steuerung des Leuchtmittels selber, um zu verhindern, dass zum Beispiel ein Leuchtmittel, das bereits einen Teil seiner Lebensdauer hinter sich hat, bei einem Einsatz in einer weiteren Maschine als neu bewertet wird. Hierdurch kennt die Leuchtmitteleinheit ihre eigene Betriebsdauer, unabhängig von der Maschine, in der es eingesetzt wird.

Bauteile, wie beispielsweise die UV-Einheit, werden daher durch diese zusätzlichen Anforderungen wesentlich komplexer in der Entwicklung und Zulassung, als dies für den eigentlichen Zweck ihres Einsatzes notwendig wäre.

Aus der JP 2002 272 822 A ist eine Aufbereitungsvorrichtung für chirurgische Instrumente bekannt, welche ein austauschbares Verschleißteil aufweist, das für einen Betrieb bis zu einer vorbestimmten Höchstbetriebsdauer vorgesehen ist. Die Aufbereitungsvorrichtung umfasst eine Leseeinheit, mit der verschleißteilspezifische Daten aus einem RFID-Transponder des Verschleißteils ausgelesen werden. Dieser Transponder ist an dem Verschleißteil angeordnet.

DE 10 2010 060 302 A1 offenbart ein Verfahren zur Zusammenstellung und Überwachung aus einer aus einer Mehrzahl von austauschbaren Systemkomponenten und einer Systemsteuereinheit bestehenden Systemanordnung. Dazu wird eine vorgegebene Systemanordnung ausgewählt und einzelne austauschbare Systemkomponenten zusammengestellt. Dabei werden alle austauschbaren Systemkomponenten erkannt und ihre relevanten Eigenschaften erfasst, um den gewünschten Gesamtprozess anhand dieser Information und der durch den Anwender vorgebbaren reinen biologischen/ chemischen Prozessparameter zu konfigurieren und zu steuern bzw. zu regeln.

Aus JP 2006-230491A1 ist eine Aufbereitungsmaschine für Endoskope bekannt, die ausgebildet ist, die Abschwächung von Radiowellen zu verhindern, so dass die Komplikation und Störung der Übertragung von Informationen hinsichtlich des Waschens und Desinfizierens beseitigt wird.

Der vorliegenden Erfindung liegt demgegenüber die Aufgabe zugrunde, die sichere Reinigung und Desinfektion von chirurgischen Instrumenten auch über die Lebensdauer von Verschleißteilen hinaus auf einfache Weise sicherzustellen.

Diese der Erfindung zugrunde liegende Aufgabe wird gelöst durch ein Verfahren zum Betrieb einer Aufbereitungsvorrichtung für chirurgische Instrumente, insbesondere Endoskope, wobei die Aufbereitungsvorrichtung eine Hauptsteuerung und wenigstens ein austauschbares Verschleißteil aufweist, das für einen Betrieb bis zu einer vorbestimmten Höchstbetriebsdauer vorgesehen ist, wobei mittels einer in der Aufbereitungsvorrichtung umfassten Leseeinheit verschleißteilspezifische Identifikationsdaten aus einem Transponder ausgelesen werden, der am oder im Verschleißteil angeordnet und mit dem Verschleißteil verbunden oder im Verschleißteil integriert ist, wobei nach einem Auslesen der verschleißspezifischen Identifikationsdaten ein Abgleich der verschleißspezifischen Identifikationsdaten mit einer externen Datenbank stattfindet, in der die verschleißteilspezifischen Identifikationsdaten gespeichert sind, wobei das Verfahren dadurch weitergebildet ist, dass ein Betrieb der Aufbereitungsvorrichtung nur dann aufgenommen und/oder durchgeführt wird, wenn der Abgleich mit der externen Datenbank ergeben hat, dass das Verschleißteil in der externen Datenbank aufgeführt und für den Betrieb in der Aufbereitungsvorrichtung zugelassen ist, wobei die Zulassung umfasst, dass jedes Verschleißteil nur einmal in eine einzige Aufbereitungsvorrichtung eingebaut werden kann.

Die Erfindung beruht auf dem Grundgedanken, dass reglementierte und freigegebene Ersatzteile, die erfindungsgemäßen Verschleißteile, beispielsweise Leuchtmittel für die UV-Einheit, nach ihrem Einbau durch einen Abgleich mit einer externen Datenbank auf ihre Zulässigkeit für den Einsatz in der Aufbereitungsvorrichtung hin überprüft werden können.

Dabei werden die Verschleißteile nun mit Transpondern versehen, die in geeigneter Weise miniaturisiert hergestellt werden können. So wird die Überwachung in die Hauptsteuerung der Aufbereitungsvorrichtung verlagert. Hierzu ist eine Kombination aus Transpondern an den Verschleißteilen und einer Leseeinheit für die Transponder in der Aufbereitungsvorrichtung vorgesehen, die die in der Aufbereitungsvorrichtung verbauten Verschleißteile erkennt und somit die Überwachung ermöglicht. Solche Leseeinheiten sind in modernen Aufbereitungsvorrichtungen teilweise bereits vorhanden, so dass eine Umrüstung bestehender Systeme einfach möglich ist.

Um zu verhindern, dass ein Verschleißteil aus einer Maschine ausgebaut wird und in einer anderen Maschine eingebaut wird und dort nicht als bereits gebraucht erkannt wird, ist zusätzlich erfindungsgemäß eine Anbindung an eine externe Datenbank vorgesehen, die Informationen über freigegebene Verschleißteile enthält, so dass eine Freigabe und Inbetriebnahme nur dann erfolgt, wenn ein Abgleich mit der externen Datenbank ergeben hat, dass dies auch zulässig ist.

Da moderne Aufbereitungsvorrichtungen bzw. deren Hauptsteuerungen ohnehin über einen Netzwerkanschluss verfügen, ist diese Maßnahme einfach zu implementieren.

Vorzugsweise wird ein Betrieb der Aufbereitungsvorrichtung nur dann aufgenommen und/oder durchgeführt, wenn der Abgleich mit der externen Datenbank ergeben hat, dass das Verschleißteil seine vorbestimmte Höchstbetriebsdauer noch nicht erreicht hat.

In einer vorteilhaften Weiterbildung ist in der Hauptsteuerung wenigstens ein Zähler integriert, mit dem die Betriebsdauer des Verschleißteils überwacht wird und sind in der externen Datenbank die bisherige Betriebsdauer, eine Höchstbetriebsdauer und/oder eine restliche zulässige Betriebsdauer gespeichert. Die Implementierung eines Zählers in der Hauptsteuerung erfolgt im Regelfall softwaregestützt und ist somit einfach implementierbar, wobei ein eigener Zähler am Verschleißteil erfindungsgemäß entfällt.

Die über die Betriebsdauer in der externen Datenbank und gegebenenfalls in einer internen Datenbank der Hauptsteuerung gespeicherten Daten hängen von der Implementierung ab. So kann die gesammelte bisherige Betriebsdauer in der externen Datenbank gespeichert sein, zusammen mit der zulässigen Höchstbetriebsdauer, mit der die bisherige Betriebsdauer beim Abgleich verglichen wird, oder es kann, insbesondere zusätzlich zur Höchstbetriebsdauer, eine restliche zulässige Betriebsdauer in der externen Datenbank gespeichert sein und/oder an die Hauptsteuerung der Aufbereitungsvorrichtung übermittelt werden. In diesem letzteren Fall ist das Zählen als Countdown der Betriebsdauer implementiert, und die Hauptsteuerung benötigt die Information über die zulässige Höchstbetriebsdauer des Verschleißteils nicht.

Vorzugsweise beinhaltet der Abgleich ein Aktualisieren der Daten zur Betriebsdauer des Verschleißteils in der externen Datenbank, insbesondere unter Protokollierung eines Zeitpunkts des Abgleichs.

Damit wird sichergestellt, dass auch die externe Datenbank aktuelle Daten über den Betriebsdauerstatus der Verschleißteile aufweist. Die Protokollierung eines Zeitpunkts des Abgleichs, der alternativ auch der Zeitpunkt der Abfrage in der Aufbereitungsvorrichtung sein kann, stellt einen Zeitstempel oder Datumsstempel dar, so dass über einen Datenabgleich mit der Maschinensteuerung oder Hauptsteuerung die Aktualität der Daten sichergestellt werden kann. Hierdurch werden der mehrfache Einsatz desselben Bauteils und auch der Einsatz nicht freigegebener Verschleißteile sicher ausgeschlossen.

Zusätzlich wird eine Rückverfolgbarkeit der verwendeten Verschleißteile ermöglicht.

In einer bevorzugten Weiterbildung ist vorgesehen, dass nach einem Austausch eines, insbesondere verbrauchten, Verschleißteils gegen ein, insbesondere baugleiches, neues oder neueres Verschleißteil in einer Aufbereitungsuntereinheit der Zähler in der Hauptsteuerung für das Verschleißteil zurückgesetzt wird und das neue oder neuere Verschleißteil nur in Betrieb genommen wird, wenn der Abgleich mit der externen Datenbank ergibt, dass das neue oder neuere Verschleißteil in der externen Datenbank vorhanden ist und noch nicht in Betrieb genommen wurde oder seine bisherige Betriebsdauer die Höchstbetriebsdauer noch nicht erreicht hat, wobei insbesondere im letzteren Fall der Zähler auf die bisherige Betriebsdauer gesetzt wird. Im Falle eines Countdowns entspricht das Nichterreichen der Höchstbetriebsdauer dem Vorhandensein einer restlichen zulässigen Betriebsdauer. Auf diese Weise wird sichergestellt, dass jedes Verschleißteil nur einmal in eine Aufbereitungsvorrichtung eingebaut werden kann und dort bis zum Erreichen ihrer Höchstbetriebsdauer betrieben wird. Nach Erreichen der Höchstbetriebsdauer eines Verschleißteils kann der Eintrag in der externen Datenbank entweder gelöscht werden, so dass der Transponder des Verschleißteils bei einem neuen Verschleißteil und zurückgesetztem Zähler erneut einsetzbar ist, oder der Eintrag in der externen Datenbank bleibt erhalten, um dieses Verschleißteil mit diesem Transponder als endgültig nicht mehr einsetzbar zu kennzeichnen. Zusätzlich kennzeichnet in diesem Fall das Erreichen der Höchstbetriebsdauer bzw. ein Ablauf der restlichen zulässigen Betriebsdauer das weitere Betreiben des Verschleißteils als unzulässig.

In vorteilhaften Alternativen wird der Abgleich bei jeder Inbetriebnahme der Aufbereitungsvorrichtung ausgeführt oder nur dann, wenn sich die Identifikationsdaten des wenigstens einen Verschleißteils geändert haben. In der ersten Alternative findet eine immer wiederkehrende Überwachung durch Abgleich mit der externen Datenbank statt. In der zweiten Alternative findet der Abgleich nur bei Erkennen eines neu eingesetzten Verschleißteils zur Identifikation statt. In diesem Fall muss nach anfänglicher Übermittlung der Höchstbetriebsdauer, der bisherigen Betriebsdauer und/oder einer restlichen zulässigen Betriebsdauer durch die externe Datenbank die Hauptsteuerung die Einhaltung der zulässigen Höchstbetriebsdauer überwachen. Hierfür verfügt sie üblicherweise über eine interne Datenbank, die laufend oder regelmäßig aktualisierte Daten zu dem oder den Verschleißteilen enthält.

Weiterhin werden in der zweiten Alternative vorzugsweise spätestens nach einem Austausch eines Verschleißteils die jüngsten das Verschleißteil betreffenden Daten an die externe Datenbank übermittelt und diese damit aktualisiert. Die externe Datenbank enthält vorzugsweise zusätzlich in den Datensätzen zu jedem Verschleißteil auch Daten über wenigstens die Aufbereitungsvorrichtung, in der das jeweilige Verschleißteil zuletzt eingesetzt war.

Vorteilhafterweise wird in der externen Datenbank zu jedem Verschleißteil eine Betriebshistorie geführt, in der zu jedem Eintrag ein Datumsstempel, die Aufbereitungsvorrichtung, die bisherige Betriebsdauer und/oder gegebenenfalls weitere Daten gespeichert werden.

Mit dieser Betriebshistorie lässt sich das Betriebsleben der Verschleißteile nachvollziehen, so dass Rückschlüsse auf die Belastbarkeit der Verschleißteile ebenso wie auf deren Verwendung möglich sind. Daraus lassen sich die Verschleißteile und deren Einsatz optimieren. Weitere Daten können von der Art des Verschleißteils abhängen, beispielsweise Füllstände von Zusatzmittelbehältern, Betriebsparameter für die Verschleißteile, Anzahlen von Schaltzyklen von Leuchtmitteln oder elektrischen Bauteilen usw.

Vorzugsweise erfolgt das Auslesen des Transponders während einer Wartung und/oder bei jeder Inbetriebnahme der Aufbereitungsvorrichtung und/oder zu Beginn, während oder nach jedem Aufbereitungsvorgang in der Aufbereitungsvorrichtung.

Mit dem Auslesen des Transponders wird sichergestellt, dass das Verschleißteil nicht ausgetauscht wurde, so dass die Betriebsdauer des Aufbereitungsvorgangs diesem Verschleißteil zugerechnet werden kann, oder, falls ein neues Verschleißteil eingesetzt wurde, bestimmt werden kann, ob dieses hierfür zugelassen ist und weiter verwendet werden darf.

Die Betriebsdauer des Verschleißteils wird vorzugsweise über eine Betriebszeitdauer oder einen kumulierten Volumenfluss eines bei Aufbereitungen anfallenden Fluidstromes gezählt. Die Betriebszeitdauer ist beispielsweise bei UV-Leuchtmitteln die geeignete Wahl der Betriebsdauer, während bei Filtern oder ähnlichem ein kumulierter Volumenfluss die Abnutzung des Filters in einigen Fällen besser beschreibt. Ferner kann auch eine Anzahl von zugelassenen Schaltzyklen eines Verschleißteils, beispielsweise eines Leuchtmittels, definiert sein und überwacht werden.

Die der Erfindung zugrundeliegende Aufgabe wird auch durch ein System zum Betrieb wenigstens einer Aufbereitungsvorrichtung für chirurgische Instrumente, insbesondere Endoskope, wobei die Aufbereitungsvorrichtung eine Hauptsteuerung und wenigstens ein austauschbares Verschleißteil aufweist, das für einen Betrieb bis zu einer vorbestimmten Höchstbetriebsdauer vorgesehen ist, gelöst, wobei das wenigstens eine Verschleißteil einen Transponder aufweist, der am oder im Verschleißteil angeordnet und mit dem Verschleißteil verbunden oder im Verschleißteil integriert ist, wobei die wenigstens eine Aufbereitungsvorrichtung eine Leseeinheit aufweist, mittels derer verschleißteilspezifische Identifikationsdaten aus dem Transponder auslesbar sind, und das System dadurch fortgebildet ist, dass die Hauptsteuerung eingerichtet ist, mit einer externen Datenverarbeitungsanlage mit einer externen Datenbank verbunden oder verbindbar zu sein, in der die verschleißteilspezifischen Identifikationsdaten, und insbesondere bisherigen Betriebsdauern, Höchstbetriebsdauern und/oder restlichen zulässigen Betriebsdauern, gespeichert oder speicherbar sind, wobei die Hauptsteuerung ferner dazu eingerichtet ist, nach einem Auslesen der verschleißteilspezifischen Identifikationsdaten einen Abgleich der verschleißteilspezifischen Identifikationsdaten mit der externen Datenbank durchzuführen, in der die verschleißteilspezifischen Identifikationsdaten gespeichert sind, wobei das System zur Durchführung des zuvor beschriebenen erfindungsgemäßen Verfahrens ausgebildet und eingerichtet ist.

Damit weist das System auch die gleichen Eigenschaften, Merkmale und Vorteile auf wie das erfindungsgemäße Verfahren.

Das System ist skalierbar, wobei vorzugsweise die externe Datenbank mit mehreren Aufbereitungsvorrichtungen verbunden oder verbindbar ist. Damit ist es möglich zu überwachen, ob ein Verschleißteil möglicherweise, beispielsweise durch einen Bedienfehler, in eine weitere Aufbereitungsvorrichtung eingesetzt wird.

Wenn vorzugsweise die externe Datenverarbeitungsanlage mit der externen Datenbank bei einem Hersteller der Aufbereitungsvorrichtungen, einem Hersteller der Verschleißteile oder in einer Einrichtung mit mehreren Aufbereitungsvorrichtungen angeordnet ist, so lassen sich unterschiedlich weite Betriebsbereiche von Aufbereitungsvorrichtungen und Verschleißteilen überwachen. So kann die Datenverarbeitungsanlage mit der externen Datenbank beispielsweise in einer Klinik mit mehreren Aufbereitungsvorrichtungen eingesetzt werden. Sie kann auch beim Hersteller der Verschleißteile oder der Aufbereitungsvorrichtungen selbst angesiedelt sein, so dass auch beispielsweise mehrere Kliniken hieran angeschlossen sein können. Ebenfalls denkbar ist die Aufstellung der Datenbank bei einem Zwischenhändler, der ein Netz von Aufbereitungsvorrichtungen und Verschleißteilen betreibt. So kann die Qualität der eingesetzten Verschleißteile sichergestellt werden.

Besonders bevorzugt sind die Transponder als RFIDs ausgebildet. Als Verschleißteil kommen beispielsweise vorzugsweise Leuchtmittel, insbesondere UV-Leuchtmittel, oder Filter in Frage.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: ein erfindungsgemäßes System in schematischer Darstellung und
- Fig. 2: ein erfindungsgemäßes Verfahren in schematischer Darstellung.

In den Zeichnungen sind jeweils gleiche oder gleichartige Elemente und/oder Teile mit denselben Bezugsziffern versehen, so dass von einer erneuten Vorstellung jeweils abgesehen wird.

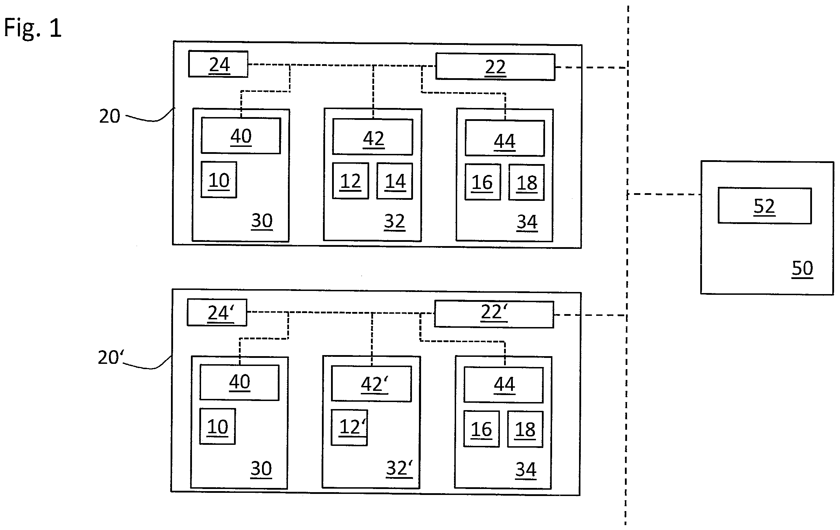

Figur 1 stellt schematisch ein erfindungsgemäßes System ausschnittsweise dar. Das System umfasst in diesem Fall wenigstens zwei Aufbereitungsvorrichtungen 20, 20' für nicht dargestellte chirurgische Instrumente, insbesondere Endoskope. Jede Aufbereitungsvorrichtung 20, 20' umfasst mehrere Verschleißteile 10, 12, 12', 14, 16, 18, die verschiedenen Funktionen bei der Reinigung und Desinfektion von chirurgischen Instrumenten dienen. So können beispielsweise die Verschleißmittel 10 UV-Leuchtmittel sein, Verschleißmittel 12, 14 und alternativ 12' Behälter mit verschiedenen desinfektionswirksamen Flüssigkeiten, während Verschleißmittel 16 und 18 beispielsweise Filter oder wärmeerzeugende Elemente darstellen können. Die Aufbereitungsvorrichtungen 20, 20' umfassen jeweils eine eigene Hauptsteuerung 22, 22', die die verschiedenen Aufbereitungsvorgänge steuern und die Verschleißteile 10, 12, 12', 14, 16, 18 bezüglich ihrer Lebensdauern bzw. Betriebsdauern überwachen. Hierzu weisen die erfindungsgemäßen Aufbereitungsvorrichtungen 20, 20' jeweils eine Leseeinheit 24, 24' auf, die Daten aus Transpondern (nicht dargestellt) an den Verschleißteilen 10, 12, 12', 14, 16, 18 auslesen, um deren Identität festzustellen. Bei den Transpondern kann es sich um RFIDs handeln.

Optional können die Aufbereitungsvorrichtungen 20, 20', wie dargestellt, Aufbereitungsuntereinheiten 30, 32 bzw. 32' und 34 aufweisen, wobei die Anzahl von jeweils drei Aufbereitungsuntereinheiten in den Aufbereitungsvorrichtungen 20, 20' willkürlich gewählt ist und nicht einschränkend aufzufassen ist. So kann eine Aufbereitungsuntereinheit 30 beispielsweise eine Flüssigkeitsdesinfektionseinheit auf UV-Basis darstellen, während andere Aufbereitungsuntereinheiten 32 bzw. 32' und 34 beispielsweise Spüleinheiten, Trocknungseinheiten etc. sein können. Jede der Aufbereitungsuntereinheiten 30, 32, 32', 34 kann optional eine Untereinheitssteuerung 40, 42, 42', 44 aufweisen, die mit der Hauptsteuerung 22, 22' jeweils verbunden ist und die Aufbereitungsvorgänge, die von der Hauptsteuerung 22, 22' angefordert werden, ausführen und steuern.

Innerhalb der Aufbereitungsvorrichtungen 20, 20' sind die verschiedenen elektronischen Komponenten über gestrichelt dargestellte Datenverbindungen miteinander verbunden.

Nach außen sind die Hauptsteuerungen 22, 22' der Aufbereitungsvorrichtungen 20, 20' über eine weitere Datenverbindung, insbesondere das Internet, die ebenfalls gestrichelt dargestellt ist, mit einer externen Datenverarbeitungsanlage 50 verbunden, die eine externe Datenbank 52 umfasst, in der Daten, unter anderem Identifikationsdaten und bisherige Betriebsdaten von Verschleißteilen, umfasst, die für den Betrieb mit den Aufbereitungsvorrichtungen 20, 20' zugelassen und gegebenenfalls schon eingesetzt sind, unabhängig davon, ob die zulässige Betriebshöchstdauer bereits erreicht ist oder nicht. Jede Hauptsteuerung 22, 22' ist ausgebildet, ihre Zähler zusammen mit den Identifikationsdaten für die Verschleißteile 10, 12, 12', 14, 16, 18 mit den in der Datenbank 52 gespeicherten Daten zu vergleichen und die Steuerung der Aufbereitung bzw. der Aufbereitungsvorrichtung 20, 20' entsprechend den Abgleichergebnissen anzupassen.

In vereinfachten Versionen können die Aufbereitungsuntereinheiten entfallen und die Aufbereitung direkt durch die Hauptsteuerung 22, 22' übernommen werden, die somit die Aufgaben der Aufbereitungsuntereinheiten und insbesondere der Untereinheitssteuerungen 40, 42, 42', 44 übernehmen.

Wie ebenfalls in Figur 1 angedeutet ist, können noch weitere Aufbereitungsvorrichtungen an die externe Datenbank 52 angeschlossen sein. Dies wird dadurch kenntlich gemacht, dass die gestrichelten Datenverbindungen nach oben und nach unten zu weiteren logischen Einheiten, d.h. weiteren Aufbereitungsvorrichtungen, die nicht dargestellt sind, laufen.

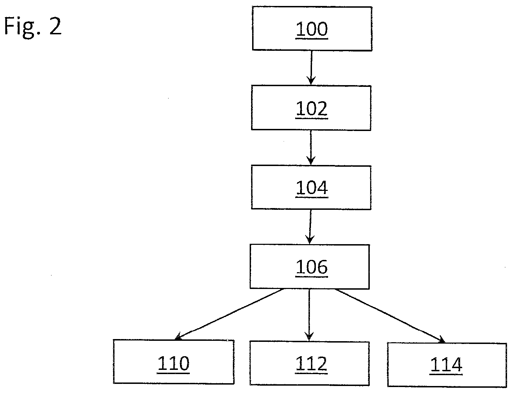

Figur 2 zeigt ein Beispiel eines erfindungsgemäßen Verfahrens. In einem (optionalen) Verfahrensschritt 100 wird ein Verschleißteil in einer Aufbereitungsvorrichtung, beispielsweise gemäß Figur 1, durch ein neues Verschleißteil, das baugleich ist oder die gleiche Funktion hat, ersetzt.

Im zwingend erfolgenden Verfahrensschritt 102 wird bei der Inbetriebnahme beispielsweise der Aufbereitungsvorrichtung mittels der Leseeinheit 24 ein RFID bzw. ein Transponder am Verschleißteil ausgelesen, um die Identität des Verschleißteils zu erfassen.

In einem Verfahrensschritt 104 wird anschließend ein Abgleich mit einer externen Datenbank 52 durchgeführt und evaluiert, ob es sich um ein bereits im Betrieb befindliches oder um ein neues Verschleißteil handelt, oder ob es vielmehr gar nicht eingesetzt werden darf.

In Verfahrensschritt 106 wird anschließend entschieden, welche Handlung vorzunehmen ist. Im Fall, dass es sich um ein nach Datenbankauskunft zulässiges neues Verschleißteil handelt, wird im Verfahrensschritt 110 der Verschleißteilbetriebsdauerzähler auf Null gesetzt und gegebenenfalls eine angeforderte Aufbereitung durchgeführt. Im alternativen Fall, dass der Verfahrensschritt 100 entfallen ist und kein neues Verschleißteil eingesetzt worden ist, wird erkannt, dass das Verschleißteil nicht ausgetauscht worden ist. Wenn die Höchstbetriebsdauer des Verschleißteils noch nicht erreicht ist, wird der Aufbereitungsvorgang durchgeführt, ansonsten wird eine Meldung ausgegeben, dass das Verschleißteil zu ersetzen ist.

In dem alternativen Verfahrensschritt 114 wurde erkannt, dass das neu eingesetzte Verschleißteil entweder nicht in der externen Datenbank 52 vorhanden ist, also auch nicht zugelassen ist, oder es sich nicht um ein neues Verschleißteil handelt, so dass ebenfalls eine Fehlermeldung ausgegeben wird.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein, sofern sie sich im Schutzumfang der anhängigen Ansprüche bewegen. Im Rahmen der Erfindung sind Merkmale, die mit "insbesondere" oder "vorzugsweise" gekennzeichnet sind, als fakultative Merkmale zu verstehen.

### Bezugszeichenliste

- 10: Verschleißteil
- 12, 12': Verschleißteil
- 14: Verschleißteil
- 16: Verschleißteil
- 18: Verschleißteil
- 20, 20': Aufbereitungsvorrichtung
- 22, 22': Hauptsteuerung
- 24, 24': Leseeinheit
- 30: Aufbereitungsuntereinheit
- 32, 32': Aufbereitungsuntereinheit
- 34: Aufbereitungsuntereinheit
- 40: Untereinheitssteuerung
- 42, 42': Untereinheitssteuerung
- 44: Untereinheitssteuerung
- 50: externe Datenverarbeitungsanlage
- 52: externe Datenbank
- 100: Verfahrensschritt
- 102: Verfahrensschritt
- 104: Verfahrensschritt
- 106: Verfahrensschritt
- 110: Verfahrensschritt
- 112: Verfahrensschritt
- 114: Verfahrensschritt

## Patentansprüche

1. Verfahren zum Betrieb einer Aufbereitungsvorrichtung (20, 20') für chirurgische Instrumente, insbesondere Endoskope, wobei die Aufbereitungsvorrichtung (20, 20') eine Hauptsteuerung (22, 22') und wenigstens ein austauschbares Verschleißteil (10, 12, 12', 14, 16, 18) aufweist, das für einen Betrieb bis zu einer vorbestimmten Höchstbetriebsdauer vorgesehen ist, wobei mittels einer in der Aufbereitungsvorrichtung (20, 20') umfassten Leseeinheit (24, 24') verschleißteilspezifische Identifikationsdaten aus einem Transponder ausgelesen werden, der am oder im Verschleißteil (10, 12, 12', 14, 16, 18) angeordnet und mit dem Verschleißteil (10, 12, 12', 14, 16, 18) verbunden oder im Verschleißteil (10, 12, 12', 14, 16, 18) integriert ist, wobei nach einem Auslesen der verschleißteilspezifischen Identifikationsdaten ein Abgleich der verschleißteilspezifischen Identifikationsdaten mit einer externen Datenbank (52) stattfindet, in der die verschleißteilspezifischen Identifikationsdaten und verschleißteilspezifische Betriebsdaten gespeichert sind
**dadurch gekennzeichnet, dass** ein Betrieb der Aufbereitungsvorrichtung (20, 20') nur dann aufgenommen und/oder durchgeführt wird, wenn der Abgleich mit der externen Datenbank ergeben hat, dass das Verschleißteil (10, 12, 12', 14, 16, 18) in der externen Datenbank aufgeführt und für den Betrieb in der Aufbereitungsvorrichtung (20, 20') zugelassen ist, wobei die Zulassung umfasst, dass jedes Verschleißteil nur einmal in eine einzige Aufbereitungsvorrichtung eingebaut werden kann.

2. Verfahren nach Anspruch 1, **dadurch gekennzeichnet, dass** ein Betrieb der Aufbereitungsvorrichtung (20, 20') nur dann aufgenommen und/oder durchgeführt wird, wenn der Abgleich mit der externen Datenbank ergeben hat, dass das Verschleißteil (10, 12, 12', 14, 16, 18) seine vorbestimmte Höchstbetriebsdauer noch nicht erreicht hat.

3. Verfahren nach Anspruch 1 oder 2, **dadurch gekennzeichnet, dass** in der Hauptsteuerung (22, 22') wenigstens ein Zähler integriert ist, mit dem die Betriebsdauer des Verschleißteils (10, 12, 12', 14, 16, 18) überwacht wird und in der externen Datenbank die bisherige Betriebsdauer, eine Höchstbetriebsdauer und/oder eine restliche zulässige Betriebsdauer gespeichert sind.

4. Verfahren nach Anspruch 3, **dadurch gekennzeichnet, dass** der Abgleich ein Aktualisieren der Daten zur Betriebsdauer des Verschleißteils (10, 12, 12', 14, 16, 18) in der externen Datenbank (52) beinhaltet, insbesondere unter Protokollierung eines Zeitpunkts des Abgleichs.

5. Verfahren nach Anspruch 3 oder 4, **dadurch gekennzeichnet, dass** nach einem Austausch eines, insbesondere verbrauchten, Verschleißteils (10, 12, 12', 14, 16, 18) gegen ein, insbesondere baugleiches, neues oder neueres Verschleißteil (10, 12, 12', 14, 16, 18) in einer Aufbereitungsuntereinheit der Zähler in der Hauptsteuerung (22, 22') für das Verschleißteil (10, 12, 12', 14, 16, 18) zurückgesetzt wird und das neue oder neuere Verschleißteil (10, 12, 12', 14, 16, 18) nur in Betrieb genommen wird, wenn der Abgleich mit der externen Datenbank (52) ergibt, dass das neue oder neuere Verschleißteil (10, 12, 12', 14, 16, 18) in der externen Datenbank (52) vorhanden ist und noch nicht in Betrieb genommen wurde oder seine bisherige Betriebsdauer die Höchstbetriebsdauer noch nicht erreicht hat, wobei insbesondere im letzteren Fall der Zähler auf die bisherige Betriebsdauer gesetzt wird.

6. Verfahren nach einem der Ansprüche 1 bis 5, **dadurch gekennzeichnet, dass** der Abgleich bei jeder Inbetriebnahme der Aufbereitungsvorrichtung (20, 20') ausgeführt wird oder nur dann, wenn sich die Identifikationsdaten des wenigstens einen Verschleißteils (10, 12, 12', 14, 16, 18) geändert haben.

7. Verfahren nach einem der Ansprüche 1 bis 6, **dadurch gekennzeichnet, dass** in der externen Datenbank (52) zu jedem Verschleißteil (10, 12, 12', 14, 16, 18) eine Betriebshistorie geführt wird, in der zu jedem Eintrag ein Datumsstempel, die Aufbereitungsvorrichtung, die bisherige Betriebsdauer und/oder gegebenenfalls weitere Daten gespeichert werden.

8. Verfahren nach einem der Ansprüche 1 bis 7, **dadurch gekennzeichnet, dass** das Auslesen des Transponders während einer Wartung und/oder bei jeder Inbetriebnahme der Aufbereitungsvorrichtung (20, 20') und/oder zu Beginn, während oder nach jedem Aufbereitungsvorgang in der Aufbereitungsvorrichtung (20, 20') erfolgt.

9. Verfahren nach einem der Ansprüche 1 bis 8, **dadurch gekennzeichnet, dass** die Betriebsdauer des Verschließteils (10, 12, 12', 14, 16, 18) über eine Betriebszeitdauer oder einen kumulierten Volumenfluss eines bei Aufbereitungen anfallenden Fluidstroms gezählt wird.

10. System zum Betrieb wenigstens einer Aufbereitungsvorrichtung (20, 20') für chirurgische Instrumente, insbesondere Endoskope, wobei die Aufbereitungsvorrichtung (20, 20') eine Hauptsteuerung (22, 22') und wenigstens ein austauschbares Verschleißteil (10, 12, 12', 14, 16, 18) aufweist, das für einen Betrieb bis zu einer vorbestimmten Höchstbetriebsdauer vorgesehen ist, wobei das wenigstens eine Verschleißteil (10, 12, 12', 14, 16, 18) einen Transponder aufweist, der am oder im Verschleißteil (10, 12, 12', 14, 16, 18) angeordnet und mit dem Verschleißteil (10, 12, 12', 14, 16, 18) verbunden oder im Verschleißteil (10, 12, 12', 14, 16, 18) integriert ist, wobei die wenigstens eine Aufbereitungsvorrichtung (20, 20') eine Leseeinheit (24, 24') aufweist, mittels derer verschleißteilspezifische Identifikationsdaten aus dem Transponder auslesbar sind, **dadurch gekennzeichnet, dass** die Hauptsteuerung (22, 22') eingerichtet ist, mit einer externen Datenverarbeitungsanlage (50) mit einer externen Datenbank (52) verbunden oder verbindbar zu sein, in der die verschleißteilspezifischen Identifikationsdaten gespeichert oder speicherbar sind, wobei die Hauptsteuerung (22, 22') ferner dazu eingerichtet ist, nach einem Auslesen der verschleißteilspezifischen Identifikationsdaten einen Abgleich der verschleißteilspezifischen Identifikationsdaten mit der externen Datenbank (52) durchzuführen, in der die verschleißteilspezifischen Identifikationsdaten und verschleißteilspezifische Betriebsdaten gespeichert sind, wobei das System zur Durchführung eines Verfahrens nach einem der Ansprüche 1 bis 9 ausgebildet und eingerichtet ist.

11. System nach Anspruch 10, **dadurch gekennzeichnet, dass** die externe Datenbank (52) mit mehreren Aufbereitungsvorrichtungen (20, 20') verbunden oder verbindbar ist.

12. System nach Anspruch 10 oder 11, **dadurch gekennzeichnet, dass** die externe Datenverarbeitungsanlage (50) mit der externen Datenbank (52) bei einem Hersteller der Aufbereitungsvorrichtungen, einem Hersteller der Verschleißteile oder in einer Einrichtung mit mehreren Aufbereitungsvorrichtungen angeordnet ist.

13. System nach einem der Ansprüche 10 bis 12, **dadurch gekennzeichnet, dass** die Transponder als RFIDs ausgebildet sind.

14. System nach einem der Ansprüche 10 bis 13, **dadurch gekennzeichnet, dass** das Verschleißteil (10, 12, 12', 14, 16, 18) als Leuchtmittel, insbesondere als UV-Leuchtmittel, oder als Filter ausgebildet ist.

## Claims

1. A method for operating a reconditioning device (20, 20') for surgical instruments, in particular endoscopes, wherein the reconditioning device (20, 20') has a main controller (22, 22') and at least one replaceable wear part (10, 12, 12', 14, 16, 18), which is provided for operating up to a predetermined maximum operating duration, wherein, by means of a reading unit (24, 24') comprised in the reconditioning device (20, 20'), wear part-specific identification data is read out from a transponder, which is arranged on or in the wear part (10, 12, 12', 14, 16, 18) and is connected to the wear part (10, 12, 12', 14, 16, 18) or integrated into the wear part (10, 12, 12', 14, 16, 18), wherein, after the wear part-specific identification data is read out, the wear part-specific identification data is compared with an external database (52), in which the wear part-specific identification data and wear part-specific operating data is stored, **characterized in that** operation of the reconditioning device (20, 20') is only commenced and/or performed when the comparison with the external database has found that the wear part (10, 12, 12', 14, 16, 18) is listed in the external database and is approved for operation in the reconditioning device (20, 20'), wherein the approval comprises that each wear part can only be installed once in a single reconditioning device.

2. The method according to claim 1, **characterized in that** operation of the reconditioning device (20, 20') is only commenced and/or performed when the comparison with the external database has found that the wear part (10, 12, 12', 14, 16, 18) has not yet reached its predetermined maximum operating duration.

3. The method according to claim 1 or 2, **characterized in that** at least one counter is integrated into the main controller (22, 22') and monitors the operating duration of the wear part (10, 12, 12', 14, 16, 18), and the previous operating duration, a maximum operating duration, and/or a remaining permissible operating duration are stored in the external database.

4. The method according to claim 3, **characterized in that** the comparison includes updating the data on the operating duration of the wear part (10, 12, 12', 14, 16, 18) in the external database, in particular logging a point in time of the comparison.

5. The method according to claim 3 or 4, **characterized in that**, after an, in particular used, wear part (10, 12, 12', 14, 16, 18) is replaced with an, in particular identical, new or newer wear part (10, 12, 12', 14, 16, 18) in a reconditioning subunit, the counter in the main controller (22, 22') for the wear part (10, 12, 12', 14, 16, 18) is reset and the new or newer wear part (10, 12, 12', 14, 16, 18) is only brought into operation when the comparison with the external database (52) finds that the new or newer wear part (10, 12, 12', 14, 16, 18) is present in the external database (52) and has not yet been brought into operation or its previous operating duration has not yet reached the maximum operating duration, wherein in particular in the latter case the counter is set to the previous operating duration.

6. The method according to one of claims 1 to 5, **characterized in that** the comparison is carried out upon each startup of the reconditioning device (20, 20') or only when the identification data of the at least one wear part (10, 12, 12', 14, 16, 18) has changed.

7. The method according to one of claims 1 to 6, **characterized in that** an operating history is kept in the external database (52) for each wear part (10, 12, 12', 14, 16, 18), in which a date stamp, the reconditioning device, the previous operating duration, and/or, if applicable, additional data are stored for each entry.

8. The method according to one of claims 1 to 7, **characterized in that** the transponder is read out during maintenance and/or at each startup of the reconditioning device (20, 20') and/or at the beginning of, during, or after each reconditioning process in the reconditioning device (20, 20').

9. The method according to one of claims 1 to 8, **characterized in that** the operating duration of the wear part (10, 12, 12', 14, 16, 18) is counted using an operating time duration or a cumulative volume flow of a fluid flow arising during reconditioning.

10. A system for operating at least one reconditioning device (20, 20') for surgical instruments, in particular endoscopes, wherein the reconditioning device (20, 20') has a main controller (22, 22') and at least one replaceable wear part (10, 12, 12', 14, 16, 18), which is provided for operation up to a predetermined maximum operating duration, wherein the at least one wear part (10, 12, 12', 14, 16, 18) has a transponder, which is arranged on or in the wear part (10, 12, 12', 14, 16, 18) and is connected to the wear part (10, 12, 12', 14, 16, 18) or integrated into the wear part (10, 12, 12', 14, 16, 18), wherein the at least one reconditioning device (20, 20') has a reading unit (24, 24'), by means of which wear part-specific identification data can be read out from the transponder, **characterized in that** the main controller (22, 22') is configured to be connected or connectable to an external data processing system (50) with an external database (52), in which the wear part-specific identification data is stored or can be stored, wherein the main controller (22, 22') is also configured, after the wear part-specific identification data is read out, to perform a comparison of the wear part-specific identification data with the external database (52) in which the wear part-specific identification data and wear part-specific operating data is stored, wherein the system is designed and configured to perform a method according to one of claims 1 to 9.

11. The system according to claim 10, **characterized in that** the external database (52) is connected or connectable to multiple reconditioning devices (20, 20').

12. The system according to claim 10 or 11, **characterized in that** the external data processing system (50) with the external database (52) is arranged at a manufacturer of the reconditioning devices, a manufacturer of the wear parts or in a device with multiple reconditioning devices.

13. The system according to one of claims 10 to 12, **characterized in that** the transponders are designed as RFIDs.

14. The system according to one of claims 10 to 13, **characterized in that** the wear part (10, 12, 12', 14, 16, 18) is designed as an illuminant, in particular as a UV illuminant, or as a filter.

## Revendications

1. Procédé pour faire fonctionner un dispositif de traitement (20, 20') pour des instruments chirurgicaux, en particulier des endoscopes, le dispositif de traitement (20, 20') présentant une unité principale de commande (22, 22') et au moins une pièce d'usure remplaçable (10, 12, 12', 14, 16, 18) qui est prévue pour un fonctionnement sur une durée de fonctionnement maximale prédéterminée, des données d'identification spécifiques aux pièces d'usure étant lues à partir d'un transpondeur au moyen d'une unité de lecture (24, 24') comprise dans le dispositif de traitement (20, 20'), qui est agencée sur ou dans la pièce d'usure (10, 12, 12', 14, 16, 18) et qui est reliée à la pièce d'usure (10, 12, 12', 14, 16, 18) ou qui est intégrée à la pièce d'usure (10, 12, 12', 14, 16, 18), une comparaison des données d'identification spécifiques à la pièce d'usure avec une base de données externe (52) ayant lieu après une lecture des données d'identification spécifiques à la pièce d'usure, pendant laquelle sont mémorisées les données d'identification spécifiques à la pièce d'usure et les données de fonctionnement spécifiques à la pièce d'usure.
**caractérisé en ce qu'**un fonctionnement du dispositif de traitement (20, 20') n'est déclenché et/ou effectué que si la comparaison avec la base de données externe a montré que la pièce d'usure (10, 12, 12', 14, 16, 18) est répertoriée dans la base de données externe et est homologuée pour un fonctionnement dans le dispositif de traitement (20, 20'), l'autorisation comprenant le fait que chaque pièce d'usure ne peut être installée qu'une seule fois dans un seul dispositif de traitement.

2. Procédé selon la revendication 1, **caractérisé en ce qu'**un fonctionnement du dispositif de traitement (20, 20') n'est déclenché et/ou effectué que si la comparaison avec la base de données externe a montré que la pièce d'usure (10, 12, 12', 14, 16, 18) n'a pas encore atteint sa durée de fonctionnement maximale prédéterminée.

3. Procédé selon la revendication 1 ou la revendication 2, **caractérisé en ce qu'**au moins un compteur est intégré dans l'unité principale de commande (22, 22'), au moyen duquel la durée de fonctionnement de la pièce d'usure (10, 12, 12', 14, 16, 18) est surveillée, et **en ce que** sont enregistrées dans la base de données externe la durée de fonctionnement jusqu'à présent, une durée de fonctionnement maximale et/ou une durée de fonctionnement restante autorisée.

4. Procédé selon la revendication 3, **caractérisé en ce que** la comparaison comprend une mise à jour dans la base de données externe (52) des données relatives à la durée de fonctionnement de la pièce d'usure (10, 12, 12', 14, 16, 18), en particulier en consignant un moment de la comparaison.

5. Procédé selon la revendication 3 ou la revendication 4, **caractérisé en ce qu'**après un remplacement d'une pièce d'usure (10, 12, 12', 14, 16, 18), en particulier usagée, par une pièce d'usure (10, 12, 12', 14, 16, 18), neuve ou plus récente, en particulier de construction identique, dans une sous-unité de traitement, le compteur est remis à zéro dans l'unité principale de commande (22, 22') pour la pièce d'usure (10, 12, 12', 14, 16, 18) et la pièce d'usure neuve ou plus récente (10, 12, 12', 14, 16, 18) n'est mise en service que si la comparaison avec la base de données externe (52) montre que la pièce d'usure neuve ou plus récente (10, 12, 12', 14, 16, 18) est présente dans la base de données externe (52) et n'a pas encore été mise en service ou que sa durée de fonctionnement jusqu'à présent n'a pas encore atteint la durée de fonctionnement maximale, le compteur étant, en particulier dans ce dernier cas, réglé sur la durée de fonctionnement jusqu'à présent.

6. Procédé selon l'une des revendications 1 à 5, **caractérisé en ce que** la comparaison est effectuée à chaque mise en service du dispositif de traitement (20, 20') ou uniquement lorsque les données d'identification de ladite au moins une pièce d'usure (10, 12, 12', 14, 16, 18) ont changé.

7. Procédé selon l'une des revendications 1 à 6, **caractérisé en ce que**, dans la base de données externe (52), un historique de fonctionnement est conservé pour chaque pièce d'usure (10, 12, 12', 14, 16, 18), dans lequel sont enregistrés, pour chaque entrée, un horodatage, le dispositif de traitement, la durée de fonctionnement jusqu'à présent et/ou éventuellement d'autres données.

8. Procédé selon l'une des revendications 1 à 7, **caractérisé en ce que** la lecture du transpondeur est effectuée lors d'une maintenance et/ou à chaque mise en service du dispositif de traitement (20, 20') et/ou au début, pendant ou après chaque opération de traitement dans le dispositif de traitement (20, 20').

9. Procédé selon l'une des revendications 1 à 8, **caractérisé en ce que** la durée de fonctionnement de la pièce d'usure (10, 12, 12', 14, 16, 18) est comptée sur la base d'une durée de fonctionnement ou d'un débit volumique cumulé d'un flux de fluide généré lors des opérations de conditionnement.

10. Système pour faire fonctionner au moins un dispositif de traitement (20, 20') pour des instruments chirurgicaux, en particulier des endoscopes, le dispositif de traitement (20, 20') présentant une unité principale de commande (22, 22') et au moins une pièce d'usure remplaçable (10, 12, 12', 14, 16, 18) qui est prévue pour un fonctionnement sur une durée de fonctionnement maximale prédéfinie, ladite au moins une pièce d'usure (10, 12, 12', 14, 16, 18) présentant un transpondeur qui est agencé sur ou dans la pièce d'usure (10, 12, 12', 14, 16, 18) et qui est relié à la pièce d'usure (10, 12, 12', 14, 16, 18) ou qui est intégré à la pièce d'usure (10, 12, 12', 14, 16, 18), ledit au moins un dispositif de traitement (20, 20') présentant une unité de lecture (24, 24') au moyen de laquelle des données d'identification spécifiques aux pièces d'usure sont aptes à être lues à partir du transpondeur, **caractérisé en ce que** l'unité principale de commande (22, 22') est agencée de façon à être reliée ou à être apte à être reliée à une installation (50) de traitement de données externe avec une banque de données externe (52), dans laquelle les données d'identification spécifiques aux pièces d'usure sont mémorisées ou sont aptes à être mémorisées, l'unité principale de commande (22, 22') étant en outre conçue pour, après une lecture des données d'identification spécifiques aux pièces d'usure, effectuer une comparaison des données d'identification spécifiques aux pièces d'usure avec la base de données externe (52), dans laquelle sont mémorisées les données d'identification spécifiques aux pièces d'usure et les données de fonctionnement spécifiques aux pièces d'usure, le système étant conçu et agencé pour mettre en œuvre un procédé selon l'une des revendications 1 à 9.

11. Système selon la revendication 10, **caractérisé en ce que** la base de données externe (52) est reliée ou apte à être reliée à plusieurs dispositifs de traitement (20, 20').

12. Système selon la revendication 10 ou la revendication 11, **caractérisé en ce que** l'unité externe (50) de traitement de données comprenant la base de données externe (52) est située chez un fabricant des dispositifs de traitement, un fabricant des pièces d'usure ou dans un établissement comprenant plusieurs dispositifs de traitement.

13. Système selon l'une des revendications 10 à 12, **caractérisé en ce que** les transpondeurs sont sous la forme de RFID.

14. Système selon l'une des revendications 10 à 13, **caractérisé en ce que** la pièce d'usure (10, 12, 12', 14, 16, 18) est conçue sous la forme d'un moyen d'éclairage, en particulier d'un moyen d'éclairage UV, ou d'un filtre.
